Europäisches Patentamt

European Patent Office

Office européen des brevets

(11) Publication number: **0 360 433**
**A1**

## EUROPEAN PATENT APPLICATION

(21) Application number: 89308680.1

(22) Date of filing: 25.08.89

(51) Int. Cl.5: **C07K 3/08 , A61K 39/44 , A61K 47/00 , G01N 33/543 , //A61K39/395,A61K37/02, C07K7/40**

(30) Priority: 26.08.88 GB 8820377

(43) Date of publication of application:
28.03.90 Bulletin 90/13

(84) Designated Contracting States:
AT BE CH DE ES FR GB GR IT LI LU NL SE

(71) Applicant: **Offord, Robin Ewart**
**65, route d'Ornex**
**CH-1239 Collex-Bossy(CH)**

(72) Inventor: **Offord, Robin Ewart**
**65 Route d'Ornex**
**CH-1249 Collex Bossy(CH)**
Inventor: **Rose, Keith**
**7 Chemin de la Boisserette**
**CH-1208 Geneve(CH)**

(74) Representative: **Hallybone, Huw George et al**
**CARPMAELS AND RANSFORD 43**
**Bloomsbury Square**
**London WC1A 2RA(GB)**

(54) Protein derivatives and a process for their preparation.

(57) Protein derivatives of formula (1)

A-CO-N(R$^1$)CH$_2$-Z-R$^2$     (1)

wherein A-CO is the residue of a protein or peptide A-CO$_2$H (especially where -CO$_2$H is the C-terminal carboxyl group of the protein or peptide); R$^1$ is a hydrogen atom or an alkyl, aryl or aralkyl group; Z$^1$, which may be present or absent is a spacer group; R$^2$ is a group -C(OH)(R$^3$)CH$_2$NHR$^4$ or -C(R$^3$)O (where R$^3$ and R$^4$ which may be the same or different, is each a hydrogen atom or an alkyl, aryl or aralkyl group); with the proviso that when A-CO is a residue of insulin, R$^1$ is other than a hydrogen atom; and the salts thereof. The compounds may be prepared by a condensation reaction,, especially in the presence of an enzyme capable of catalysing the formation of a peptide bond. The compounds may be used to form protein conjugates in which a group of interest such as another protein or peptide or an effector or reporter group is coupled to the protein through a -N(R$^1$)CH$_2$-Z$^1$-C(OH)(R$^3$)CH$_2$N = or -N(R$^1$)-CH$_2$Z$^1$-CH = group.

## PROTEIN DERIVATIVES AND A PROCESS FOR THEIR PREPARATION

Field of the Invention

This invention relates to protein derivatives, to a process for their preparation and to their use in the preparation of novel protein conjugates which are of use in medicine. More particularly the invention relates to amino-alcohol derivatives and a;dehyde of proteins and to their preparation, especially by enzyme catalysed reverse proteolysis.

Background to the Invention

There is considerable interest in medicine in the use of protein conjugates, for either therapy or diagnosis. The conjugate generally comprises a protein, such as an antibody, to which is attached an effector or a reporter group, for example a cytotoxic agent or a group capable of being radioactively labelled.

In general, attachment of effector or reporter groups to proteins is made by group-specific, but not regio-specific reactions (e.g. by iodination of tyrosine residues, or acylation of lysine residues). As a result, even mono-reacted material is in general itself heterogeneous, being a mixture of species each with one group attached to one of several residues of the type involved in the modification reaction.

For a protein conjugate intended for use in medicine, much is to be gained from the homogeneity of the product of site specific attachment of a group of interest, in contrast to the product obtained by the non-regiospecific procedures described above. Thus, the advantages of site-specific modification of antibodies of the IgG type have been reported [Murayama, A et al, Immunochemistry 15, 523-528, (1978); Rodwell, J.D et al Proc. Natl. Acad. Sci. USA 83, 2632-2636, (1986) and European Patent Specification No. 88695]. In this case, regiospecificity was achieved by oxidation of the carbohydrate of IgG molecules, followed by reductive alkylation. The success of such an approach depends upon the existence of appropriate glycosylation, so it is not generally applicable to other classes of polypeptide, and since even some IgG molecules posses more than one site of glycosylation, the approach may not always be site specific.

The specificity of proteases working in reverse has been exploited to fix activating groups exclusively at the carboxyl terminus of polypeptides [Offord, R.E. and Rose, K. in Protides of the Biological Fluids (H. Peeters, ed.) Pergamon, Oxford pp35-38, (1986); Offord, R.E. et al in Peptides 1986 (D. Theodoropoulos, ed.) W. de Gruyter, Berlin, pp279-281, (1986); Rose, K et al in Peptides 1986 (ibid), pp219-222, (1986); Rose, K. et al, Biochem. J. 249, 83-88, (1988); and European Patent Specification No. 243929]. In a subsequent chemical reaction an effector or reporter group may be directed specifically to the activated group of the modified polypeptide. (It is not usually possible or desirable to fix enzymatically the group of interest directly to the polypeptide chain for reasons of enzyme specificity and the large excess of amino component generally required to drive the enzymatic coupling to high yield). The specificity of the conjugation reaction between the effector or reporter group and the activated polypeptide may be achieved by chemical complementarity of the reacting groups in the effector or reporter molecule and the polypeptide, or, in the case of the effector or reporter molecule being itself a polypeptide fragment, by conformational assistance.

Activating groups which are aromatic aldehydes, aliphatic aldehydes or aromatic amines which have been described for use in protease directed reactions of the above type. Conjugation was achieved via formation and reduction of a Schiff base (reductive alkylation) and specificity was obtained by virtue of the relative reactivities of aromatic versus aliphatic amines at low pH.

We have now found that it is possible to use enzyme catalysed reverse proteolysis to attach amino-alcohol groups specifically to the carboxyl termini of proteins. No side chain protection is required and the coupling advantageously takes place under very mild conditions and in aqueous solutions where denataration of the protein can be kept to a minimum. Amino-alcohol groups are not normally found in proteins and site specific conjugations may be effected by directing appropriately modified effector or reporter groups to the hydrazide group when these latter have been attached enzymically to the protein. The resulting conjugate requires no further treatment, in contrast to previous reports of site specific attachment (Murayama, A et al, Rodwell, J.D et al, Offord, R.E. and Rose, K., Offord, R.E. et al, ibid) where reductive stabilisation of a Schiff base is necessary. Since such reduction can be responsible for the irreversible formation of unwanted inter- and intramolecular links, higher specificity may thus be obtained using conjugation via amino-alcohol groups.

Summary of the Invention

According to one aspect of the invention we provide a compound of formula (1):

A-CO-N(R¹)CH₂-Z¹-R²     (1)

wherein

A-CO is the residue of a protein or peptide A-CO₂H;

R¹ is a hydrogen atom or an alkyl, aryl or aralkyl group;

Z¹, which may be present or absent, is a spacer group;

R² is a group -C(OH)(R³)CH₂NHR⁴ (where R³ and R⁴, which may be the same or different, is each a hydrogen atom or an alkyl, aryl or aralkyl group) or -C(R³)O; with the proviso that when A-CO- is a residue of insulin, R¹ is other than a hydrogen atom; and the salts thereof.

In the compounds of formula (1), the residue of a protein or peptide ACO₂H represented by A-CO- may be the residue of any naturally occurring, synthetic or semi-synthetic protein or peptide, including proteins and peptides produced by hybridoma and recombinant DNA techniques. Particular examples include residues of plasma proteins, such as immunoglobulins, (for example polyclonal, monoclonal and recombinant antibodies and fragments thereof), fibrinogen, albumin and transferrin, hormones for example steroid hormones such as oestrogens, insulin, erythropoietin and somatotropin, lymphokines such as interleukin 1 or interleukin 2, cytokines such as tumour necrosis factor, industrially and therapeutically useful enzymes such as tissue plasminogen activator and enzyme inhibitors such as tissue inhibitor of metalloproteinases.

When the group R¹, R³ or R⁴, in compounds of formula (1) is an alkyl group it may be for example a C₁₋₆alkyl group such as a methyl or ethyl group. Examples of aryl groups represented by R¹, R³ or R⁴ are C₆₋₁₂aryl groups, for example phenyl groups. Aralkyl groups represented by R¹, R³ or R⁴ include ArC₁₋₃alkyl, e.g. phenC₁₋₃alkyl such as benzyl or phenethyl.

When Z¹ in compounds of formula (1) is a spacer group it may be for example an optionally substituted aliphatic hydrocarbyl chain optionally interrupted by one or more heteroatoms selected from -O-or -S- or one or more -N(R⁵)- (where R⁵ is a hydrogen atom or a C₁₋₆alkyl group), cycloaliphatic or aromatic groups.

Thus, in particular, Z¹ may represent an optionally substituted straight or branched C₁₋₁₀alkylene, C₂₋₁₀alkenylene or C₂₋₁₀alkynylene chain, optionally interrupted by one or more heteroatoms selected from -O- or -S- or one or more -N(R⁵) [e.g. -NH- or -N(CH₃)-] cycloaliphatic (e.g. C₃₋₈ cycloalkylene such as cyclohexylene) or aromatic (e.g. C₆₋₁₂arylene, such as phenylene) groups.

Particular examples of Z¹ include -CH₂-, -(CH₂)₂-, -CH₂OCH₂-, -CH₂SCH₂-, -(CH₂)₃- or --

(CH₂)₄-

Salts of compounds of formula (1) include salts with acids and bases, for example acid addition salts such as hydrochlorides or hydrobromides, or alkali metal salts such as sodium or potassium salts.

In general, the A-CO- group in compounds of formula (1) may be the residue of a protein or peptide A-CO₂H wherein -CO₂H is any carboxyl group present in the protein or peptide. Preferably, however, A-CO-in compounds of formula (1) is the residue of a protein or peptide A-CO₂H wherein -CO₂H is the C-terminal carboxyl group of the protein or peptide.

A preferred group of compounds of formula (1) are those wherein A-CO- is the residue of a plasma protein such as fibrinogen or an immunoglobulin or a fragment thereof, a hormone, such as insulin, a cytokine such as tumour necrosis factor, or a therapeutically useful enzyme such as tissue plasminogen acitvator.

In particular, A-CO- is preferably an immunoglobulin or a fragment thereof. The immunoglobulin or immunoglobulin fragment may in general belong to any immunoglobulin class. Thus, for example, it may be an antibody belonging to an immunoglobulin M class or, in particular, an antibody belonging to an immunoglobulin G class. The antibody, or fragment thereof, may be of animal, for example mammalian origin and may be for example of murine, rat or human origin. It may be a natural antibody or a fragment thereof, or if desired, a recombinant antibody or antibody fragment, i.e. an antibody or antibody fragment which has been produced using recombinant DNA techniques.

Particular recombinant antibodies or antibody fragments include, (1) those having an antigen binding site at least part of which is derived from a different antibody, for example those in which the hypervariable or complementarity determining regions of one antibody have been grafted into the variable framework regions of a second, different antibody (as described in European Patent Specification No. 239400); (2) recombinant antibodies or fragments wherein non-Fv sequences have been substituted by non-Fv sequences from other, different antibodies (as described in European Patent Specifications Nos. 171496, 173494 and 194276); or (3) recombinant antibodies or fragments possessing substantially the structure of a natural immunoglobulin but wherein the hinge region has a different number of cysteine residues from that found in the natural immunoglobulin, or wherein one or more cysteine residues in a surface pocket of the recombinant antibody or fragment is is the place of another amino acid residue present in the natural immunoglobulin (as described in International Patent Application Nos. PCT/GB 88/00730 and PCT/GB 88-00729 respectively).

The antibody or antibody fragment may be of polyclonal, or, preferably, monoclonal origin. It may be specific for any number of antigenic determinants, but is preferably specific for one. The antigenic determinant may be any hapten or an antigenic determinant associated with animals, e.g. humans, [for example normal animal tissue or organ cell-associated antigens, tumour cell-associated antigens (for example oncofetal antigens such as carcinoembryonic antigen or alphafetoprotein, placental antigens such as chorionic gonadotropin and placental alkaline phosphatase, and prostate antigens such as prostatic acid phosphatase and prostate specific antigen) and antigens associated with components of body fluids such as fibrin or platelets], viruses, bacteria and fungi.

Antibody fragments include for example fragments derived by proteolytic cleavage of a whole antibody, such as $F(ab')_2$, $Fab'$ or $Fab$ fragments, or fragments obtained by recombinant DNA techniques, for example Fv fragments (as described in International Patent Application No. PCT/GB 88-00747).

$R^1$, and, when present, $R^3$ and $R^4$ in compounds of formula (1) are preferably hydrogen atoms or alkyl, especially methyl groups. Compounds of formula (1) in which $R^1$, and when present, $R^3$ and $R^4$ is each a hydrogen atom are particularly preferred.

When $Z^1$ in compounds of formula (1) is present, it is preferably a $C_{1-10}$alkylene chain, optionally interrupted by one or more -O- or -S- atoms or -$N(R^5)$- groups.

A particularly useful group of compounds according to the invention has the formula (1a):

$A-CO-N(R^1)CH_2-R^2$     (1a)

wherein A-CO-, $R^1$, and $R^2$, are as defined for formula (1) with the proviso that when A-CO- is a residue of insulin, $R^1$ is other than a hydrogen atom; and the salts thereof.

Especially useful compounds of this type have the formula (1b):

$A-CO-NHCH_2CH(OH)CH_2NH_2$     (1b)

wherein A-CO- is as defined for formula (1) but is not an insulin residue, and the salts thereof; or the formula (1c):

$A-CO-NHCH_2CHO$     (1c)

wherein A-CO- is as defined for formula (1) but is not an insulin residue, and the salts thereof.

Compounds of formulae (1a), (1b) and (1c) wherein A-CO- is the residue of a protein or peptide $A-CO_2H$ wherein -$CO_2H$ is the C-terminal carboxyl group of the protein or peptide are particularly preferred. Compounds of this type wherein A-CO- is the residue of an immunoglobulin or a fragment thereof are especially useful.

Compounds of formula (1) may be prepared by the following processes wherein A-CO-, $R^1$ $R^2$ and $Z^1$ are as defined for formula (1) except where otherwise specified.

Thus according to a further aspect of the invention we provide a process for the preparation of a compound of formula (1) wherein $R^2$ is a group -$C(OH)(R^3)CH_2NHR^4$ which comprises condensing a compound of formula (2):

$A-CO_2H$     (2)

or an activated derivative thereof with a compound of formula (3):

$R^1NHCH_2-Z^1C(OH)(R^3)CH_2NHR^4$     (3)

in a solvent, for example an aqueous solvent, at a suitable temperature, for example around ambient temperature.

In a particular aspect of the above general process according to the invention we provide a process for the preparation of a compound of formula (1) wherein A-CO- is the residue of a protein or peptide $A-CO_2H$ and -$CO_2H$ is the C-terminal carboxyl group of the protein or peptide, which comprises condensing a compound of formula (2) with a compound of formula (3) in the presence of an enzyme capable of catalysing the formation of a peptide bond.

Suitable enzymes for use in this aspect of the invention include proteases, in particular endopeptidases, such as serine proteinases, e.g. trypsin or chymotrypsin, or lysyl endopeptidase, or exopeptidases, for example carboxypeptidases, such as carboxypeptidase Y.

In general, the reaction may be performed under advantageously mild conditions selected to favour the condensation of the compound of formula (2) with the compound of formula (3). The exact conditions used will depend on the enzyme in use. Thus, for example, using trypsin as the enzyme the reaction may be effected in the presence of a solvent, especially an aqueous solvent, for example water or an aqueous organic solvent, for example aqueous dimethylsulphoxide aqueous dimethylacetamide or aqueous butane-1,4-diol, at an appropriate pH, for example in the range pH4-8, at a suitable temperature, for example around ambient temperature.

Other enzymes may be employed in the process using the same or similar conditions, the exact nature of which may be determined empirically using appropriate small-scale tests in accordance with conventional practice prior to carrying out the condensation reaction.

Compounds of formula (1) wherein $R^2$ represents a group -$C(R^3)O$ may be prepared by oxidation of a corresponding compound of formula (1), for example using an oxidising agent such as periodic acid in a solvent such as ethylene glycol at a suitable temperature, for example, around ambient temperature.

Salts of compounds of formula (1) may be prepared using standard procedures, for example by reacting a compound of formula (1) with an acid or base in an aqueous solvent.

Proteins or peptides of formula (2) are readily available or may be obtained using standard procedures.

Intermediates of formula (3) are either known compounds or may be prepared from known starting materials using methods analogous to those used for the preparation of the known compounds.

Compounds of formula (1) are useful for the preparation of protein conjugates in which a protein is linked to a further molecule of interest, for example another protein or a peptide, or an effector, or reporter molecule, the linkage comprising a -N(R$^1$)-CH$_2$-Z$^1$-C(OH)(R$^3$)CH$_2$N= or -N(R$^1$)CH$_2$Z$^1$-CH= group.

Thus according to a further aspect of the invention we provide a compound of formula (1) for use in the preparation of a protein conjugate.

According to yet another aspect of the invention we provide a protein conjugate wherein a protein is linked to another protein or a peptide or an effector or reporter molecule, characterised in that the linkage comprises a -N(R$^1$)CH$_2$-Z$^1$-C(OH)(R$^3$)-CH$_2$N= or -N(R$^1$)CH$_2$Z$^1$-CH= group.

In conjugates of this type the groups R$^1$, R$^3$, and Z$^1$ are as defined for formula (1), and the various preferences expressed for certain types of these groups in connection with formula (1) are also to be understood to apply to protein conjugates according to this aspect of the invention.

Particular protein conjugates which may be prepared using compounds of formula (1) generally include compounds of formula (4):

A-CO-N(R$^1$)CH$_2$-Z$^1$-Y-R    (4)

wherein A-CO-, R$^1$ and Z$^1$ are as defined for formula (1), R is a protein or peptide or an effector or reporter group and Y is a linking group formed by coupling the -C(OH)(R$^3$)CH$_2$NHR$^4$ or -C(R$^3$)O group in compounds of formula (1) with a reactive group in the group R.

Particular examples of compounds of formula (4) include compounds of formula (5):

A-CO-N(R$^1$)CH$_2$-Z$^1$-CH=N-R    (5)

wherein A-CO-, R$^1$, Z$^1$, and R are as just defined.

In compounds of formulae (4) and (5), R may be for example a protein or peptide as defined above for A-CO-, or an effector or a reporter group. Effector groups include, for example any physiologically active substance, antibacterial, antiviral, or antifungal compound. Particular physiologically active substances include antineoplastic agents, including cytotoxic and cytostatic agents, toxins, hormones, anti-inflammatory compounds, and substances active as cardiovascular, e.g. fibrinolytic, and central nervous system, agents. Reporter groups include for example a group which is detectable by analytical means in vitro and/or in vivo, such as a group capable of being radioactively labelled, a fluorescent group or a group capable of being monitored by NMR or ESR spectroscopy.

It will be appreciated that in the formation of the compounds of formulae (4) and (5) the starting material R must possess a group capable of reacting with the hydrazide group of the compound of formula (1). Such groups may be readily identified, and include, especially, aldehyde groups. The appropriate group may already be present in the starting material or, when necessary, may be introduced using conventional techniques for example as described in the Intermediates and Example herein.

The following Intermediates and Examples illustrate the invention:

Intermediate 1

Coupling of 1,3-diamino-2-propanol to desAla$^{B30}$insulin (DAI)

1,3-diamino-2-propanol (94mg-Fluka) was dissolved in 2ml of a mixture of butane-1,4-diol (Fluka; 9 volumes) and distilled in water (1 volume). The apparent pH (uncorrected glass electrode) was adjusted to a measured 6.8 by addition of acetic acid (Fluka, puriss). This acidification must be performed with care to ensure adequate mixing of the rather viscous solution and to allow time for the relatively slow response of the electrode in such a solvent. DAI (30mg) was dissolved in 1.5ml of the above solution and 3mg TPCK-treated bovine trypsin (Worthington) was added dissolved in 30µl water. The enzyme solution was rapidly and thoroughly mixed in. After incubation for 4h at 22°C, the reaction mixture was worked up as described for couplings of other nucleophiles (Rose et al., Biochem. J. 211 1983 671-676; acidification, gel filtration on G50 fine in 1% acetic acid, lyophilization of the peak of insulin derivatives, now free of solvent, reagents and trypsin). Yield of crude material was approximately 28mg. Analysis of the crude material by electrophoresis on cellulose acetate at pH8 showed a small amount of remaining DAI and a large amount (about 70%) of material migrating very slowly towards the anode, as would a compound having two net negative charges less than DAI (see Rose et al., Biochem. J. 211 1983 671-676). This is consistent with a product of structure DAI-NHCH$_2$CH(OH)CH$_2$NH$_2$. Analysis by electrophoresis on cellulose acetate at low pH (10% acetic acid) showed the product to migrate faster than insulin towards the cathode, consistent with its possessing an extra positive charge at low pH. Upon analysis by reversed phase HPLC the product was found to be more hydrophilic (elute earlier) than DAI, consistent with a structure having an extra positive charge at low pH. The coupling yield was about 70%. DAI-NHCH$_2$CH(OH)CH$_2$NH$_2$ was purified by HPLC on System 1:

## System 1:

Beckman Gold system. The column was Machery Nagel Nucleosil 300A 5μm C8 25cm x 4mm i.d. operated at 0.6ml/minute. Solvent A was 0.3M ammonium sulphate and solvent B was 0.3M ammonium sulphate in 35% acetonitrile. The two solvents were made up using stock solutions of 3M ammonium sulphate which had been adjusted to an indicated pH of 2 (glass electrode) by careful addition of concentrated sulphuric acid. Detection was at 254nm.

Fractions were collected manually and, after dilution with water, protein was recovered on a C18 SepPak (Waters) used according to the manufacturer's instructions [methanol wash, equilibration with 0.1% trifluoroacetic acid (TFA), sample application after prior dilution with an equal volume of water, washing with 0.1% TFA/20% acetonitrile, elution with 0.1% TFA/40% acetonitrile.] Solvents were removed in a vacuum centrifuge (SpeedVac Concentrator, Savant) with the heater switched off.

The product was shown to be pure by electrophoresis on cellulose acetate at pH8 and in 10% acetic acid. Digestion of DAI-NHCH$_2$CH(OH)-CH$_2$NH$_2$ with Armillaria mellea protease (Rose et al, in Peptides 1984, Ragnarsson, U. Ed. pp 235-238, Almqvist and Wiksell International, Stockholm) liberated des(Lys$^{B29}$Ala$^{B30}$)insulin, identified by electrophoresis on cellulose acetate at pH8 and by HPLC. In view of the above data, and the method of synthesis, the product was assigned the structure DAI-NHCH$_2$CH(OH)CH$_2$NH$_2$, where the diaminopropanol group is attached to the alpha-carboxyl group of Lys$^{B29}$.

## Intermediate 2

### Oxidation of DAI-NHCH$_2$CH(OH)CH$_2$NH$_2$

To 1.8ml of a solution of DAI-NHCH$_2$CH(OH)-CH$_2$NH$_2$ (prepared and purified as for Intermediates 1; 1.1mg/ml in 1% ammonium bicarbonate solution, pH 8.3) was added 5.8μl ethylene glycol (Fluka) with thorough mixing. 200μl periodic acid solution (1.96mg/ml in water) were added with thorough mixing (final concentrations DAI-NHCH$_2$CH(OH)CH$_2$NH$_2$, 0.19mM; glycol 57.6mM; periodate 0.96mM). After incubation in the dark at 22°C for 30 minutes, the mixture was analysed by HPLC System 1. DAI-NHCH$_2$CH(OH)CH$_2$NH$_2$ was transformed almost quantatively into a later eluting substance, consistent with loss of a positive charge. The produce was isolated by preparative HPLC on System 1 and recovered by SepPak and vacuum centrifugation as described in Example 1. The product was identified as the expected DAI-NHCH$_2$CHO by it properties on HPLC (more hydrophobic than DAI-NHCH$_2$CH(OH)CH$_2$NH$_2$), on electrophoresis on cellulose acetate in 10% acetic acid (migrates close to insulin position, considerably slower than DAI-NHCH$_2$CH(OH)CH$_2$NH$_2$) and by reaction with aldehyde-seeking reagents.

## Example 1

### Conjugation of DAI-NHCH$_2$CHO to aminooxyacetyl-ferrioxamine

Zinc-free porcine insulin (control) and DAI-NHCH$_2$CHO were incubated at 100μM concentration in 0.1M sodium acetate buffer at pH4.6 at 22°C in the presence and absence of 500μM aminooxyacetylferrioxamine (prepared as described in European Patent Specification No. 243929). At intervals, 10μl aliquots were analyzed by HPLC, the results showing that insulin does not react with the aminooxy compound, that DAI-NHCH$_2$CHO is stable in the absence of aminooxy compound that the aminooxy compound is stable and that DAI-NHCH$_2$CHO reacts cleanly to produce a single more hydrophobic conjugate. The product was orange, stable at pH2-8 (wider range not investigated), and is the expected oxime of DAI-NHCH$_2$CHO.

## Claims

1. A compound of formula (1):
A-CO-N(R$^1$)CH$_2$-Z$^1$-R$^2$     (1)
wherein
A-CO- is the residue of a protein or peptide A-CO$_2$H;
R$^1$, is a hydrogen atom or an alkyl, aryl or aralkyl group;
Z$^1$, which may be present or absent, is a spacer group;
R$^2$ is a group -C(OH)(R$^3$)CH$_2$NHR$^4$ (where R$^3$ and R$^4$, which may be the same or different, is each a hydrogen atom or an alkyl, aryl or aralkyl group) or -C(R$^3$)O; with the proviso that when A-CO is a

residue of insulin, $R^1$ is other than a hydrogen atom; and the salts thereof.

2. A compound of formula (1a):

A-CO-N($R^1$)CH$_2$-$R^2$     (1a)

wherein

A-CO is the residue of a protein or peptide A-CO$_2$H;

$R^1$, is a hydrogen atom or an alkyl, aryl or aralkyl group;

$R^2$ is a group -C(OH)($R^3$)CH$_2$NHR$^4$ (where $R^3$ and $R^4$, which may be the same or different, is each a hydrogen atom or an alkyl, aryl or aralkyl group) or -C($R^3$)O; with the proviso that when A-CO is a residue of insulin, $R^1$ is other than a hydrogen atom; and the salts thereof.

3. A compound according to Claim 1 or Claim 2 wherein A-CO is the residue of a protein or peptide A-CO$_2$H wherein -CO$_2$H is the C-terminal carboxyl group of the protein or peptide.

4. A compound according to any of the preceding claims wherein $R^1$, $R^3$ and $R^4$ is each a hydrogen atom.

5. A compound according to any of the preceding claims wherein A-CO is the residue of an immunoglobulin or a fragment thereof.

6. A process for the preparation of a compound of formula (1):

A-CO-N($R^1$)CH$_2$-$Z^1$-$R^2$     (1)

wherein

A-CO- is the residue of a protein or peptide A-CO$_2$H;

$R^1$, is a hydrogen atom or an alkyl, aryl or aralkyl group;

$Z^1$, which may be present or absent, is a spacer group;

$R^2$ is a group -C(OH)($R^3$)CH$_2$NHR$^4$ (where $R^3$ and $R^4$, which may be the same or different, is each a hydrogen atom or an alkyl, aryl or aralkyl group); with the proviso that when A-CO is a residue of insulin, $R^1$ is other than a hydrogen atom; and the salts thereof,

which comprises condensing a compound of formula (2):

A-CO$_2$H     (2)

or an activated derivative thereof with a compound of formula (3):

$R^1$NHCH$_2$-$Z^1$C(OH)($R^3$)CH$_2$NHR$^4$     (3)

7. A process according to Claim 7 for the preparation of a compound of formula (1) wherein A-CO is the residue of a protein or peptide A-CO$_2$H and -CO$_2$H is the C-terminal carboxyl group of the protein or peptide, which comprises condensing a compound of formula (2) with a compound of formula (3) in the presence of an enzyme capable of catalyzing the formation of a peptide bond.

8. A protein conjugate wherein a protein is linked to another protein or a peptide or an effector or reporter molecule, characterised in that the link-

age comprises a -N($R^1$)CH$_2$-$Z^1$-C(OH)($R^3$)CH$_2$N = or N($R^1$)CH$_2$Z$^1$-CH = group wherein $R^1$, $R^3$ and $R^4$ which may be the same or different is each a hydrogen atom or an alkyl, aryl or aralkyl group and $Z^1$ which may be present or absent, is a spacer group.

9. A compound of formula (4):

A-CO-N($R^1$)CH$_2$-$Z^1$-Y-R     (4)

wherein

A-CO is the residue of a protein or peptide A-CO$_2$H;

$R^1$ is a hydrogen atom or an alkyl, aryl or aralkyl group;

$Z^1$, which may be present or absent, is a spacer group;

R is a protein or peptide or an effector or reporter group; and

Y is a linking group formed by coupling a -C(OH)($R^3$)CH$_2$ NHR$^4$ or -C($R^3$)O group (where $R^3$ and $R^4$ is each a hydrogen atom or an alkyl, aryl or aralkyl group) with a reactive group in the group R.

European Patent Office

# PARTIAL EUROPEAN SEARCH REPORT

which under Rule 45 of the European Patent Convention shall be considered, for the purposes of subsequent proceedings, as the European search report

Application number

EP 89 30 8680

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,Y | EP-A-0 243 929 (F. HOFFMANN-LA ROCHE & CO.) <br><br> * Whole document * | 1-9 | C 07 K 3/08 <br> A 61 K 39/44 <br> A 61 K 47/00 <br> G 01 N 33/543// <br> A 61 K 39/395 <br> A 61 K 37/02 <br> C 07 K 7/40 |
| P,Y | H. PEETERS: "Protides of the bio-logical fluids", 1986, pages 35-38, Pergamon Press; R.E. OFFORD et al.: "Press-stud protein conjugates" <br><br> * Whole article * | 1-9 | |
| P,A | PEPTIDES 1986 - Proceedings of the 19th European Peptide Symposium, Porto Carras, Chalkidiki, 31st August - 5th September 1986, pages 219-222, Walter de Gruyter & Co., Berlin, DE; <br><br> ./ | | |

**TECHNICAL FIELDS SEARCHED (Int. Cl.4)**

C 07 K 3/00
A 61 K 39/00
A 61 K 47/00
G 01 N 33/00
A 61 K 37/00
C 07 K 7/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely: 1-9

Claims not searched:

Reason for the limitation of the search:

The exact structure of $Z_1$ os not sufficiently disclosed in the claims; the search is there-fore restricted to the subject matter supported by the examples(i.e. $Z_1$ is absent).

| Place of search | Date of completion of the search | Examiner |
|---|---|---|
| The Hague | 11-12-1989 | NOVOA Y SANJURJO M.A. |

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | K. ROSE et al.: "Enzyme-assisted semisynthesis of polypeptide active esters for subsequent spontaneous coupling" -- | | |
| P,A | PEPTIDES 1986 - Proceedings of the 19th European Peptide Symposium, Porto Carras, Chalkidiki, 31st August - 5th September 1986, pages 279-281, Walter de Gruyter & Co., Berlin, DE; R.E. OFFORD et al.: "Press-stud protein conjugates" -- | | TECHNICAL FIELDS SEARCHED (Int Cl.4) |
| P,A | BIOCHEMICAL JOURNAL, vol. 249, 1988, pages 83-88, GB; K. ROSE et al.: "Enzyme-assisted semisynthesis of polypeptide active esters and their use" ------- | | |